# EUROPEAN PATENT APPLICATION

(11) **EP 3 971 753 A1**
(43) Date of publication of application: **23.03.2022**
(21) Application number: 21000216.8
(22) Date of filing: 05.08.2021
(51) Int. Cl.: G06F 21/62, G16H 10/60

(54) **METHOD FOR COLLECTING DATA ON DOGS, ACCESSING AND HANDLING THESE DATA AND EQUIPMENT FOR CARRYING OUT THIS METHOD**

(30) Priority: 07.09.2020 CZ 20200491
(71) Applicant: Nemec, Josef, 405 02 Decin (CZ)
(72) Inventor: Nemec, Josef, 405 02 Decin (CZ)
(74) Representative: Danek, Vilém

(57) **Abstract**

A method of collecting, accessing and handling data on dogs, wherein access to the data on dogs is available to the user by means of a dedicated system for processing instructions from communication means comprising user devices, depending on the user's authorization level, wherein the system comprises at least two authorization levels for processing instructions from the user, particularly in accessing and entering data stored in a database, where the first group of user roles has the highest level of authorization and is in particular authorized to enter and/or edit data in the database, and another group of user roles is authorized by the system only to ask questions and give answers, wherein within user roles there are superiority and subordination relationships. At least one user role has an exclusive position of superiority over at least one other user role that is in a superior and/or subordinate relationship to other user roles, and furthermore at least two user roles are not in a subordinate and/or superior relationship to any other user role, where at least one user role without a subordinate and/or superior relationship belongs to the first group of user roles with the highest level of authority, wherein at least one user role which is in a superior and subordinate relationship to other user roles belongs to the first group of user roles with the highest level of authorization. The device for carrying out the method according to the invention comprises remote data storage, a mobile and/or non-portable computing device and a chip which marks the animals for subsequent identification of its owner.

## Description

### Technical Field

The invention relates to a method for collecting data on dogs, accessing and handling these data, which relate in particular to dogs which are identifiable by means of, for example, a chip, or tattoos and dogs with a pedigree certificate, their owners, tests, dog shows, bonitations, diseases and defects, their descendants and ancestors, etc., and serve primarily to improve the breeding of all breeds of dogs, information about individual dogs, optimize and increase the efficiency of data collection related to all dogs and to speed up the collection and refinement of data on dogs for the FCI, interest organizations, clubs of breeders of individual breeds in individual states, natural persons, the police, veterinary administration and possibly for other entities. The invention further relates to a device for carrying out this method.

### Background Art

There are two basic groups of dogs. The first group involves dogs with a pedigree certificate - pedigreed dogs, and the second includes dogs without a pedigree certificate - non-pedigreed dogs. The pedigree certificate is hereinafter abbreviated as "PDG".

### Non-pedigreed dogs

In the group of dogs without pedigree certificates, the parents of these dogs are not known. Due to very frequent inbreeding, hereditary defects and diseases are transmitted from parents to offspring due to the fact that their ancestors are unknown.
These individuals are identifiable only by a chip.

### Registration of non-pedigreed dogs

Each dog must have a chip in the body, usually in the neck, which can be used to obtain a unique code composed of numbers and characters. This code is shown on the vaccination card.

In the case of traveling abroad, microchip marking is mandatory.

### Access to information about a non-pedigreed dog's owner:

The chip can perform its function, which consists in finding and identifying a lost animal, only on the assumption that the owner enters the animal's data into one or more of many private databases and stores it under the chip number. There exist numerous private registers, and if, for example, a dog is found, then a microchip number can be determined using a special reading device. It is necessary to try all private registers, because there is no central national dog register yet. So, it is solely the good will and interest of the finder, usually a police officer or a shelter worker, to carefully go through all possible registers and try to find the dog's owner.

Another problem is that so far there is no obligation to register the dog, so even after reading the microchip number, it is often not possible to find the dog's owner.

### Pedigreed dogs

In the group of dogs with pedigree certificates, their parents and other ancestors are known. We are certain of their origin and there is a lower risk of hereditary diseases and defects, which are often inherited due to very close inbreeding. Within the breeding rules of each breed, there are special rules for crossbreeding of individuals, where one of the factors that is monitored is the coefficient of inbreeding - abbreviated COI, i.e., the number of the same ancestors in the father and mother of the offspring. These rules are usually supervised by breeding advisers within breeders' clubs.

### Registration of dogs with pedigree certificates

All pedigreed individuals are registered in breed registers (studbooks) within each state. These studbooks are run by the national canine organization. In the Czech Republic, it is the Czech-Moravian Cynological Union - abbreviated CMCU.

This organization issues a unique number to each puppy born in the country, and it issues a new pedigree certificate in the case of the import of an individual from another state and assigns to the individual a new registration number for the territory of the home state.

It issues a pedigree certificate in the case of an individual's export to another state.

This organization registers all breeding establishments, their owners and dog owners in the country.

Through this organization, the state is a member of the international canine organization or FCI, which relates to the breeding of all breeds of dogs, sets its rules and oversees compliance with these rules.

This organization includes various breeding and training clubs, which govern the breeding and training of individual breeds.

### Access to information about the owner of a dog with PDG

There is the same problem as with dogs without PDG, because without registering the microchip number in private databases, it is not possible to identify the owner of the dog.

International Canine Federation - Federation Cynologique Internationale, abbreviated FCI, based in Belgium.

In most countries of the world, there are national canine organizations which are full members of the FCI with all the rights and obligations that ensue. These organizations are the highest organization (authority) that manages the breeding of recognized dog breeds in the country.

Powers of national canine organizations within the member states.

### Breeding of individual breeds of dogs - breeding rules

In relation to national canine organizations (in the Czech Republic it is the CMCU), breeders' clubs are the guarantors for the regularity of the breed or breeds. Breeding is only possible by means of a breeders' club, as a member of it, or in the case of a non-member, on the basis of a contract concluded with the club for the provision of breeding services. The breeders' club determines the conditions for inclusion in the breeding for its breed or breeds and has the right to create its own regulations regarding breeding. However, they must be in accordance with CMCU and FCI regulations.

### Selection of a suitable individual for mating

When searching for the ideal individual to "cover", the coefficient of inbreeding - COI is used. Based on a mathematical formula, the degree of close relatedness of the ancestors of a stud dog and brood bitch is calculated. If the COI is higher than the permitted limit, for example 1%, the union should not be carried out due to the close relationship of the individuals.

A large number of breeders' clubs recommend calculating the COI from a larger number of generations of ancestors, min. five generations.

If we have only a paper pedigree certificate of the dog's origin up to the 4th generation, it is not possible to calculate the COI with greater accuracy and so it is not possible to prevent unwanted inbreeding with a greater degree of certainty.

### Method of registration of data on dogs with PDG and their amount

Canine organizations in individual states keep studbooks.
They use various dog data management solutions in their area. These are, for example, various tables or SW programs.

Each dog born in a breeding establishment, i.e., to breeding parents with PDG, is issued a dog pedigree certificate in paper form. This paper pedigree certificate includes essential information, such as the owner of the dog, the work tests performed, and titles obtained at dog shows.

**In the case of breeding individuals,** the owner of such a dog is obliged to report newly acquired titles and tests to the canine organization that covers the breeding of dogs in the country and will enter them in its database - in the studbook.

If the dog is not a breeding individual, these data are no longer entered in the studbook.

For example, the surgical removal of defects which prevent the subsequent adoption of the dog for breeding purposes must still be entered in the paper pedigree certificate. However, no one will ever know this information again because it remains with the dog's owner.

A significant disadvantage of such a method of recording data on dogs is the voluntariness of transmission of the data on dogs to the canine organization of the country. The obligation to report new titles from dog shows and tests performed related to current breeding dogs is not enforceable and the owners of these breeding dogs do not always provide these data.

The number of organised dog shows, performance tests and the associated awarding of titles is huge and only a small amount of this information is actually registered.

However, even the data transmitted in this way remain inaccessible and can only be accessed by breeding advisers if they receive an extract from the studbook.

Attributes currently registered in the dog's pedigree:
- name of male dog/female dog
- breeding establishment where they were born
- pedigree certificate number
- parents back to 4th generation
- dog's owner and his/her address
- gender
- date of birth
- coat colour
- coat type
- dog shows
- performance tests

In each subsequent generation of the dog's ancestors, the pedigree certificate states fewer and fewer attributes, *inter alia* due to limited space on the paper form. In the last, i.e., 4th generation, it is possible to find out only the name of the ancestor, the pedigree certificate number and dog's gender.
The number of attributes and thus the usefulness of the data collected by the current procedure is insufficient.

For some, especially working and hunting breeds, it is desirable to record other attributes of dogs, such as height, weight, special abilities and skills, possible defects and diseases that prevent the adoption of the dog for breeding.

One of the biggest problems with the current data management system is not sharing information about dog diseases and defects. These are mainly hereditary defects. Therefore, male and female dogs are sometimes crossed, and their offspring have health problems that are not even known about.

The breeders' club, by means of a breeding advisor, manages the breeding of the given breed, and provides breeding services to non-members of the club. When the breeding advisor changes, the new breeding advisor usually starts from the beginning, because the essential information and experience gained by the previous breeding advisor over many years is difficult to pass on. It is just a matter of voluntariness and necessarily good relationships between breeding advisors. The situation is much more complicated in the case of the transfer of information between breeding advisers at the international level.

### Health records of dogs

Every dog must be regularly vaccinated. The carried-out vaccination, including its validity, must be recorded on a paper vaccination card. This is subsequently required for all dog shows, tests, or even for example when a man is bitten by a dog. If, in the event of a person being bitten by a dog, it is not possible to immediately check whether the dog has a valid vaccination, the bitten person must undergo painful vaccination against rabies as a precaution.

Data on diseases, operations on and medical treatment of dogs are registered only with a specific veterinarian. If the dog is forced to undergo surgery at another veterinarian, such as when on holiday abroad or in the event of a sudden serious injury outside the reach of "its" veterinarian, this information is not available.

Other disadvantages of the state of the art can be seen from the further description in the Summary of the Invention, which contains a comparison of the state of the art and the proposed method according to the invention.

Patent documents relating to the collection of data on animals are known, e.g., document CZ2019-502 called "Method of collecting data on game, accessing them and handling these data and the equipment for carrying out this method" describes a method of collecting data on game, accessing and handling these data and the equipment for carrying out this method, wherein the gathering of game data is carried out by entering of the relevant data by an authorized user according to his/her user role into a data application uploaded locally to that user's device and/or accessible via the user's device via a data network and stored in a remote data storage, wherein the data are uploaded online essentially in real time or after the off-line mode of the data application is terminated, and data are loaded to the database in the remote data storage. Access to the data on game is available to the user depending on the user's authorization level, with at least part of the data accessible to the public and part of the data secured by a username and password, where access to such protected data takes place after authorization of the authorized user in the data application, which handles the data stored in the database on the remote data storage. The management of the data on game is divided according to the level of data security and user roles, where user roles are divided according to the type of entity and its needs and/or the needs of the state administration, where this division of roles includes the following list of types: hunter, hunting manager, municipality with extended powers, region, Ministry of Agriculture, state veterinary administration, hunting ground holder, owner of hunting land and the police. The device for carrying out the method according to the invention comprises a remote data storage, a mobile and/or non-portable computing device, a locating device and/or an optical tracking device. The disadvantage of this solution is the impossibility of applying the given method for the needs set out in the present invention.

Also known is document US2015379191A1 [Samuel Johnson, US], which describes an invention that checks animal breeding records to identify the offspring of any selected ancestor and annotates information about the relationship of each offspring to the selected ancestor. This information can be compiled for multiple ancestors, thus providing a basis for discovering all descendants that represent any mixture of ancestor elements in any number of relationships to those ancestors as specified by the researcher. The solution is most useful in connection with database records, the Internet, and graphic user interfaces. Bloodline and pedigree researchers can make a comprehensive selection using the resources specified here.

Another known document is US6730023B1 [HEMOPET [US], which describes a system, methods, and apparatus for computerized database management of phenotypic health assessment and genomic mapping and genetic screening of animals. Numerous remote users can access a computer network connected to a central data processing database management resource. Appropriate fees are charged for data accessing, retrieval and security. Users can enter data on animal health, lifespan and genetic background and obtain reports on the evaluation of the phenotypic health of a particular subject or a group of animals and the genotypic characteristics of the particular subject or group of animals to which that subject belongs.

Nevertheless, none of the above systems or methods is able to achieve all of the advantages described below that are achieved by the method and apparatus of the present invention. The above-mentioned systems and methods can resolve only partial problems of the prior art.

### Summary of the Invention

The invention relates to a method of collecting data on dogs, accessing them, and handling them, wherein the data relate in particular to dogs, breeding establishments, their breed, their owners, characteristics, etc., and serves in particular to improve the breeding of all breeds of dogs, their protection, health and to speed up the collection and refinement of data on dogs for other organizations, breeders' clubs, breeding establishments, dog owners, shelters, the police, veterinarians and others.

There is a known method of collecting, accessing and handling data on dogs, where collection of the data is carried out by entering of the relevant data on dogs by an authorized user according to his/her user role into a data application uploaded locally to that user's device and/or accessible via the user's device by means of a data network and stored in a remote data storage, the data being uploaded online essentially in real-time or, after the off-line mode of the data application has ended, to a database in the remote data storage.

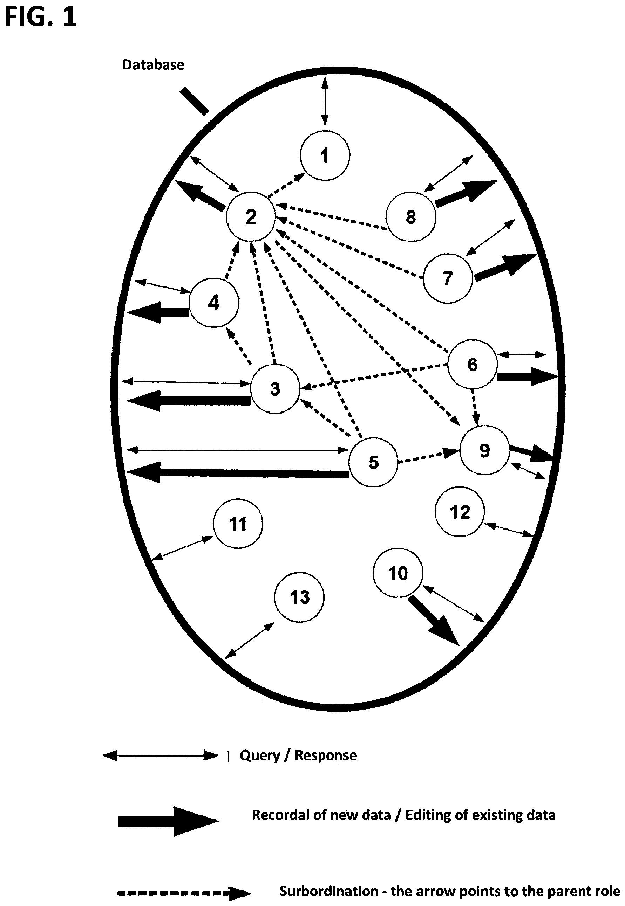

The method according to the invention, which is based on the attached scheme in Fig. 1, is characterized in that:
- access to data on dogs is available to the user by means of dedicated system processing instructions from communication means involving the user's device, depending on the user's authorization level,
- wherein the system comprises at least two levels of authorization to process instructions from the user, in particular to access data in the stored database and to enter these data into the database,
- wherein the first group of user roles with the highest level of authorization is authorized to enter and/or edit data in the database based on the authorization of the user role belonging to this group and is also authorized to ask questions and give answers through the system, which are recorded by communication tools in the database, and/or are sent directly to designated users by communication means, depending on their user role,

- where another group of user roles is only allowed to ask questions and give answers by means of the system, which are recorded by means of communication tools in the database and/or are sent directly to the designated users by communication means, depending on their user role,
- where there are superior and subordinate relationships within user roles, where the superior user role usually authorizes the requests of the subordinate user role and the subordinate user role raises authorization requests to the superior user role,
- where at least one user role, usually the entity responsible for organizing relations at the international level, which is usually an international canine organization, has an exclusive position of superiority over at least one other user role which is in a superior and/or subordinate relationship to other user roles,
- and furthermore at least two user roles are not in a subordinate and/or superior relationship to any other user role, where at least one of these user roles without a subordinate and/or superior relationship belongs to the first group of user roles with the highest level of authorization,
- wherein at least one user role, which is in a superior and subordinate relationship to other user roles, belongs to the first group of user roles with the highest level of authorization.

In a preferred embodiment, the first group of user roles with the highest level of authorization comprises at least one entity selected from groups of entities, where:
- at least one entity is responsible for the organization of cynology at the national level;
- at least one entity is responsible for providing advice on dog breeding;
- at least one entity is a breeders' club;
- at least one entity is a dog owner;
- at least one entity is the owner of a breeding establishment;
- at least one entity is responsible for assessing the appearance of dogs;
- at least one entity is responsible for assessing performance tests;
- at least one entity is responsible for monitoring and supervision of compliance with veterinary rules, and
- at least one entity is responsible for veterinary care.

In another preferred embodiment, the group of user roles without the highest authorization comprises at least one entity selected from a group of entities, where:
- at least one entity is responsible for the care of animals that are not owned by the owner or whose owner is unknown;
- at least one entity is responsible for catching and/or reporting animals that are not in the owner's possession or whose owner is unknown.

In another advantageous embodiment, the initial authorization of the user according to its user role is carried out by means of an electronic invitation from the authorized person sent to the user to its specified electronic mail address and subsequent connection via a link in the electronic invitation by entering the registration form of the data application, where the member enters his/her password to the pre-filled identification data of the member through which he/she is authorized, or the user is authorized by creating a user account through the application after personal verification of the user's identity by an authorized person or an entity authorized by it, when after user authorization the login data for the application are made available to him/her.

Even more advantageously, the entity responsible for the organization of cynology at the national level is in a superior relationship to at least one entity selected from the group comprising:
- at least one entity that is a breeders' club;
- at least one entity that is a dog owner;
- at least one entity that is the owner of a breeding establishment;
- at least one entity that is responsible for assessing the appearance of dogs;
- at least one entity that is responsible for assessing performance tests;
wherein the entity responsible for the organization of cynology at the national level is further subordinated to at least one entity selected from the group consisting of:
- at least one entity responsible for organizing relations at the international level, and
- at least one entity responsible for monitoring and supervision of compliance with veterinary rules.

In another alternative preferred embodiment, the entity responsible for providing advice in the field of dog breeding is in a superior relationship to at least one entity selected from a group comprising:
- at least one entity that is a dog owner, and
- at least one entity that is the owner of a breeding establishment;
wherein the entity responsible for providing advice in the field of dog breeding is in a subordinate relationship to at least one entity selected from the group comprising:
- at least one entity responsible for the organization of cynology at the national level, and
- at least one entity that is a breeders' club.

In another alternative preferred embodiment, the entity that owns the dog is subordinate to at least one entity selected from the group comprising:
- at least one entity responsible for the organization of cynology at the national level;
   at least one entity responsible for providing advice on dog breeding, and
- at least one entity responsible for monitoring and supervision of compliance with veterinary regulations.

In another alternative preferred embodiment, the entity that is the owner of a breeding establishment is in a subordinate relationship to at least one entity selected from the group comprising:
- at least one entity responsible for the organization of cynology at the national level;
   at least one entity responsible for providing advice on dog breeding, and
- at least one entity responsible for monitoring and supervision of compliance with veterinary regulations.

The device for carrying out the method according to the invention is characterized in that it comprises the following elements:
- remote data storage, in particular cloud data storage and/or data server;
- mobile and/or non-portable computing devices, including in particular a smartphone, tablet, and personal computer;
- a chip identifying the animals and subsequently identifying their owner;

The device includes a remote data storage accessible via a dedicated application, which can be accessed locally after installing the application on a computing device, such as a personal computer, mobile phone, or tablet, or is available online via a remote interface and the Internet. The data entered into the application can be stored in online mode, essentially in real time, or in offline mode in case a signal to the data network is not available.

The object of this invention is, inter alia, to establish online interconnection of dog breeding organizations and individuals and other organizations.

### Description of a specific embodiment and advantages of the invention

In a specific embodiment, which is characteristic of the current situation, these are the following entities:
- International Canine Federation - Federation Cynologique Internationale, abbreviated FCI
- national canine organisations of individual states
- national clubs of breeders of individual breeds
- breeding consultants
- owners of breeding establishments
- dog owners
- judges evaluating dogs' appearance
- judges evaluating dogs' performance
- veterinary authority
- veterinarians
- animal shelters
- the police
- other entities

When using the application designed to record dogs and their characteristics, ongoing information is available online, i.e., immediately and in real time.

The advantage of this solution is the creation of one large national, transnational, or global database of information about dogs, their owners and breeding establishments.

The method according to the invention provides the following advantages:

### Sale of dogs outside the original state:

Currently, when selling a dog outside the original state, the stud book worker must first issue an export PDG (export pedigree).

Only on the basis of this export PDG can the studbook worker register the dog in the new state and assign it a new PDG number.

According to this proposed method, the studbook worker would create only an electronic export PDG, a copy of which could also be printed. The studbook worker of the other state would electronically confirm and accept this report. Subsequently, an informative email would be sent to both the studbook of the original state, the former dog owner, and the new owner of the dog.

The solution would also eliminate the practice of assigning a new PDG number for each sale of a dog outside the state. At the birth of a puppy, the studbook would assign it only one - an international number, which would remain unchanged.

### Creation of an electronic pedigree of dogs

Now, in a paper PDG, one learns only a minimum of important information about a specific dog.
Due to the lack of space on the paper PDG certificate, in the first generation of ancestors you can find out only the dog's name, breeding establishment, gender, PDG number, coat colour and type, titles from dog shows and performance results. In the other generations of ancestors, due to the lack of space in the paper PDG, there is less and less information until in the fourth generation, we can only see the name of the dog, breeding establishment, pedigree number and its gender.

Many breeders' clubs are calling for a special annex to the PDG certificate, where it would be possible to find out other characteristics of the dog that are important when choosing the best individual for mating.

According to this method, thanks to the knowledge of the ancestors of individual dogs, it is possible to display in graphical form an electronic click-through pedigree to infinity. In addition to the current practice, the electronic pedigree can display all important characteristics of dogs, such as:
- Graphic highlighting of identical ancestors
- Owner (state)
- Date of birth
- Height
- Weight
- COI in several generations - e.g., in five generations.
- Results of hip dysplasia examinations.
- Number of matings
- Number of direct descendants
- etc.

After clicking on the name of any dog, regardless of its age, in the end it is possible to display all the information. This information, or limited information, could be made available to each user according to its pre-set role.

### Breeding of individual breeds of dogs and selection of the most suitable individuals for mating.

Individual breeders' clubs are in charge of the breeding of each breed by means of a breeding adviser. These breeding advisers must also provide breeding services to non-club members on the basis of a contract.

In the search for the ideal individual to "cover", the coefficient of inbreeding (COI) is used, among other things.

Based on a mathematical formula, the degree of mutual relationship between a stud dog and a brood bitch is calculated. If the COI is higher than the permitted limit, for example 1%, the union should not occur due to the close relationship of the individuals. Formula for calculating the coefficient of inbreeding Fx - **Wright's equation Fx = Σ (0.5 n1 + n2 + 1) * (1 + Fa),** where **n1** is the number of non-related generations from the sire to the common ancestor, **n2** is the number of non-related generations from the dam to the common ancestor. We refer to the non-related generation as one where there is no common ancestor. **Fa** is the inbreeding coefficient of the common ancestor which itself originated by inbreeding.

Often, particularly in small and special breeds, there is a lack of breeding individuals in the country. This is due to the non-sharing of this information at the international level and so breeding and crossbreeding are usually carried out between only a few breeding individuals in the given state.

By sharing data on breeding individuals between states, it is then easy to select a suitable individual for mating, which will lead to the desired so-called "revival of blood".

### Results from dog shows and subsequent breeding of dogs

Dog shows are occasions where special and recognized evaluators/assessors of dogs evaluate not only the appearance and special traits of dogs, but also their character (aggressivity, etc.).

Breeding rules include a list of appearance and character defects of each breed of dogs, which do not allow its subsequent breeding. If the judge finds any such defect, at present, the result is such that the dog's owner leaves the dog show, and this information is not entered in the dog's PDG.

However, the practice is such that the owner of the dog waits and then participates in another dog show, where the judge will be "less picky". Unfortunately, this is a common practice because the stakes are too high with respect to the possible sale of puppies.

According to the proposed procedure, each assessor should, if he/she finds any such defects, be obliged to enter this in the electronic PDG. The assessor is a recognized and professional person who is the only one that has the right to evaluate this. The opinion of the dog owner as a layman is not professional in this case and is often influenced by his/her personal interests in using the dog in breeding.

Then, e.g., any subsequent dispute may be addressed by the board of appeal. This ensures that any undesirable characteristics of the dog will not be transferred. It has the greatest influence on breeding quality, and it is unequivocally beneficial for the descendants' health.

Veterinarians should have the same obligation to make an entry in the electronic records of dogs in the case of any substantial information about problems having an impact on the dog's health. This particularly relates to illnesses which directly endanger the life of a dog, such as epilepsy, etc.

There would also be the possibility of a dog owner voluntarily "admitting" a defect or a disease in his/her dog which has an impact on breeding.

In the case of an electronic database, even these characteristics of dogs can be registered and undesirable crossbreeding of individuals with genetic defects would be avoided.

### Creation of the database of so-called problem litters

With knowledge of the possible defects of individuals, it is easy to find out the identity of their parents because these defects are mostly hereditary.

According to this procedure, it is easy to view all the so-called problem litters, when at least one offspring from the litter had severe health problems or defects. Thus, before an individual is recommended for mating, the breeding adviser can be assured that the descendants of the individual do not suffer from any such diseases or defect.

### Identification of puppy farms

This is currently a big problem that many states are trying to resolve. The breeding rules of each breed determine the maximum number of litters of one female dog during, for example, one year and in general during its life, as well as its minimum and maximum age for mating. At present, when the registration of litters and then the puppies is done in an outdated way, it is almost impossible to clearly display this information.

Moreover, owners of breeding establishments who are classic puppy farmers set up puppy farms in other countries so that it is not possible to trace this information at all. It is not unusual for one person to have three breeding establishments in three different states.

According to this method, it is easy to view all brood bitches and sort them according to the number of their litters and direct offspring, regardless of which breeding establishment the brood bitch is currently in. A change in the owner of the brood bitch or its sale to another state do not affect this.

Once such "breeders" have been identified, it is easy to initiate, for example, proceedings to close such a breeding establishment, to impose a fine, or to initiate other proceedings under the law of the given state.

### Repeated mating

Hereditary defects or diseases, such as epilepsy, can sometimes appear in dogs several years later. If a brood bitch is mated to one and the same stud dog several times and then, after a few years, their offspring show a hereditary defect or disease, it is a big problem because several dozen individuals are born who should not otherwise have been born at all.

Each breed has different conditions for cross mating, but it is usually allowed to mate a brood bitch with one and the same stud dog at most twice in its life.

The number of matings by a stud dog is also monitored, which is the same as with brood bitches.

It is in the best interests of the quality of breeding, the health of dogs and also in the interests of all future dog owners not to do so.

Nobody would knowingly buy a dog that has an increased risk of a serious illness or defect.

According to this method, thanks to the electronic registration of all matings and litters, it is again very easy to detect this. A change in owner or sale to another state will not mask this.

### Reporting the loss of a dog

Today, when a dog is lost, the owner of the dog usually reports this to the nearest police station, calls the nearest shelters and hopes that someone will call.

If he/she has not voluntarily registered the dog's chip number in one of the many private databases, there is little chance of finding the dog.

In case a dog is lost outside the state of origin, there is almost no chance of finding it.

According to this method, each dog is easily traceable using the chip number, which is automatically part of the information about a particular dog without the need to register it in any other private database. Any dog owner can report such a loss simply, electronically. After marking a dog in the register as lost, and a chip number check, e.g., by the police, at a shelter, at a veterinarian's, at a dog show, etc., the lost one would be immediately detected.

In this way, the number of thefts of dogs would be greatly reduced, because this would be detected at the next check of the chip number, for example at the veterinarian's.

Thanks to this method, dog owners can be traced anywhere, regardless of the country of origin.

### Creation of a global database of dogs

Today, there is no way to find out any information about specific dogs across individual countries.

According to this method, a global database of dogs, their characteristics, their owners, a database of breeding establishments and their owners would be created. This would be of great importance for breeding advisers in selecting suitable individuals for mating, for veterinarians, in detecting dog breeds, in identifying owners of lost dogs, etc.

### Electronic health card of a dog

At present, if the owner of a dog is forced to visit a veterinarian other than "his/her one", the vet cannot use information on the dog's treatment, its operations, X-ray images, medications used, etc.

The vet is forced to rely on information from the dog's owner, if one is currently available.

According to this method, the veterinarian would first find out the chip number by means of a reader and then open the dog's health card.

There he/she would find all the important information needed to choose the right treatment. It would be the veterinarian's responsibility to record all procedures and treatments, including vaccinations, validity of vaccinations, deworming, etc., in the dog's electronic health record.

### Making information on the validity of vaccinations available

Today, every compulsory vaccination is entered in a paper vaccination card and its validity is marked. If the card gets lost, these data are usually completely lost too if they are not stored by the veterinarian on his/her own computer. If, for example, a person is bitten by a dog and the dog owner is not available to prove a valid vaccination, the bitten person must undergo painful preventive vaccination against rabies.

Pursuant to this method, these data are also made available to certain user roles (police, shelters, dog show organizers, etc.) If necessary, it is easy to verify the validity of the vaccination even without a paper vaccination card.

### Creating a registry of members of breeders' clubs

At present, each breeders' club records its members in different ways. Most often they use classic .xls tables.

There are also lists of currently bred stud dogs and brood bitches within each club. These are the only records kept.

According to this method, an electronic register of members could be created with all the necessary information about the members, including address, email, phone number, etc. Each member would be entered into this database/register and have access to selected information according to his/her user role.

One important piece of information would be the ownership of a particular dog(s)or of a breeding establishment. The connection of a member to the database would take the form of, for example, an electronic invitation, which the club registrar, or another member of the club committee, would have the right to send. Another option would be to generate access data by another, higher authority.

For example, within each breeders' club, it would be possible to view basic contacts for other members of the club in a limited format (name, surname, and club function). The amount of information disclosed about the members is just a matter of agreement in the club and the GDPR.

The advantage of this method is the extensive information about dogs within a particular breeder' club which are directly owned by club members.

It is important information particularly in the case of working and hunting dog breeds, where results and awards from dog shows are not the only criteria for breeding. In these clubs, which also deal with the training of dogs and their subsequent use in practice, it is important to know how each dog works after it has passed training and testing.

For example, within the special hunting breed "blood-hound", which is a breed of hunting dog specializing in tracking shot game, it is possible to record, for example, each use of the dog to track injured game and thus determine the intensity and success of its work during its life.

The intensity of its deployment and its success in tracking shot game is one of the criteria influencing the subsequent recommendation of the breeding advisor relating to mating.

### Offering new-born puppies

According to this method, owners of breeding establishments can offer new-born puppies using this database and a special application. One will see how many puppies, of what gender, etc., are offered for sale. It will also be possible to attach a photo of the puppy.

Furthermore, it is possible for those interested in a puppy to access to its electronic click-through pedigree, where they can check important information themselves, including the coefficient of inbreeding, dog shows and performance tests of all ancestors of the puppy.

This is also usable at the international level, which can have a very positive effect on "refreshing" the blood of the breed within the state as this will expand the breeding base and reduce inbreeding.

### Connecting individual users to the database

Access is authorized and unauthorized.

**Unauthorized access** is for anybody, even for those not registered. When using this connection without registration, it is possible, for example, to see a list of all breeders' clubs, breeding establishments, breeding advisors, an overview of all dog breeds, etc.

**Authorized access,** therefore, access, e.g., by e-mail with password, which ensures access to information according to a pre-set user role.

The administrator of the entire database will create basic login information for the International Canine Federation or FCI, for individual canine organizations of each state and possibly for breeders' clubs.

National canine organizations themselves can generate these login details for breeders' clubs or for dog owners and breeding establishments.

Connection for dog owners or breeding establishments can be provided, e.g., by the breeders' club if the person is a member of this club, or by means of the administrator.

### Overview of user roles:

There can be many user roles, and each of them can have different permissions and rights as needed.

Basic overview of user roles, which can be additionally arbitrarily added, their rights can be arbitrarily modified, especially with regard to GDPR and the requirements within each state and each breeders' club.

### Basic list of possible user roles

**International Canine Federation** - **Federation Cynologique Internationale, shortened to FCI**
   - It is entitled to see all the information.
**National canine organisations of individual states** - **stud books**
   - This is the highest role at the national level of the state in question.
   - It accredits the foundation of new breeding establishments and registers them.
   - It has access to all information about dogs and their owners.
   - It makes additional connections available for breeders' clubs, owners of breeding establishments and dog owners.
   - It can register new-born dogs in breeding establishments and assigns pedigree numbers.
   - It issues export PDG.
   - It approves imports of dogs and registers them.
**National clubs of breeders of individual breeds**
   - They are entitled to see selected information about the dogs of the given breeds that they breed;
   - They keep an electronic register of members, their dogs and breeding establishments.
**Breeding advisers**
   - This role has access to a large amount of information about the dogs it needs for proper performance of its activities, i.e., to select the most suitable individual for mating.
   - It can also have the right to edit selected attributes of dogs.
**Owners of breeding establishment**
   - This role allows new litters (register newly born puppies) within a breeding establishment to be created.
   - They see an overview of all breeding individuals of the breed they breed.
   - They can offer newly born puppies through this database in a special part of the application.
**Dog owners**
   - Dog owners can see the list of all their dogs, living and already dead.
   - They can see all registered information about their own person, their dog including its health card.
   - They can modify selected data including personal contact information.
   - They can see change logs, i.e., who edited any attributes - characteristics of the dog, and when.
- **Judges assessing dogs' appearance**
   - They can see selected details related to dogs and their owners.
   - They can check who is the dog's real owner.
   - They can see the dog's valid vaccination.
   - They are entitled to enter for instance the dog's appearance and character attributes, for example, defects which forbid its breeding, e.g., crooked teeth or aggressivity. Each breed has its own breeding standard, according to which the judge, an expert on the breed, decides on breeding.
   - They have the right to record in the dog's details its titles and awards from dog shows.
**Judges assessing dogs' performance**
   - They can see selected details related to dogs and their owners.
   - They can identify the dog's real owner.
   - They can see the dog's valid vaccination.
   - They are entitled to enter, for example, the dog's character attributes, e.g., defects that forbid its breeding, e.g., aggressivity.
   - They have the right to record in the dog's details its titles and awards from dog shows.
**Veterinary administrations and veterinarians**
   - They can see selected details related to dogs and their owners.
   - They can identify the dog's real owner.
   - They can see the dog's valid vaccination.
   - They have the right to record in the dog's details all health information and to store, for example, x-ray images, etc.
**Shelters**
   - They can see selected details related to dogs and their owners.
   - They can identify the dog's real owner.
   - They can see the dog's valid vaccination.
**The police**
   - They can see selected details related to dogs and their owners.
   - They can identify the dog's real owner.
   - They can see the dog's valid vaccination.
**Other persons**
   - They can see selected details related to dogs and breeding establishments.

Data are uploaded to the remote data storage by means of an application mainly in online mode, or in case of limited data access to the network, e.g., in complicated terrain without data coverage, the data are stored in the user's local application and after logging in they are uploaded to the data network storage and synchronized.

### Device for carrying out the method according to the invention:

The device according to this invention includes the following elements:
- a remote data storage, e.g., a cloud data storage, possibly including a data server;
- mobile and/or non-portable computing devices, including in particular a smartphone, tablet, and personal computer;
- a chip used to identify animals for the subsequent identification of their owners;
- a reading device that can read the information stored on the chip.

### Login to the system

To log in to the system, the user must first open a webpage through which the necessary data are made available to all users.

For authorized access to the system, each user must enter, e.g., their email or other login name, and must add their personal password. Based on this authorized connection, the system determines which role the user has been assigned.

In the case of a request for higher data security and access to the system, access can be granted by entering only the username, with a system-generated password sent to the user in the form of, for example, an SMS to his/her phone.

Access for unregistered users does not require a username or password. In this case, the user will only have access to a limited amount of information. By means of this connection without registration, it is possible, for example, to see only a list of all breeders' clubs, breeding establishments, breeding advisors, an overview of all breeds, etc. Other data are subject to user registration.

### Setting up the role

Each user's role is set up by the system administrator or by a role superior to it. The administrator or the superior role can create a new user in the part of the system dedicated to user data management. Within this step, after clicking on the icon "Create new user", a list of mandatory and optional data/attributes that must be filled in will be displayed. The mandatory data can be, e.g., name, surname, email, and telephone number. Optional data can include, e.g., address, date of birth and others.

When creating a new user, the role is assigned to the new user, which role decides the user's rights and the amount of information that the user has the right to edit or only to see. In the same part of the system where user data are managed, the administrator or the superior role can, if necessary, change already set roles.

### The first connection of the user to the system

The administrator or the superior role can connect the user in the form of, for example, an "invitation email". The system sends an email with a link to the login webpage to the user to its predefined email. After clicking on the link in the invitation email, a webpage will open to register the new user. There the user only enters his/her personal password and will save it. This only verifies the correctness of the entered email, and the user will be identified. In the case of a request for a higher level of data security and access to the system, it is also possible to send an SMS with a generated password to the entered telephone number.

In case the password is lost or forgotten, it is possible to regenerate it. All you have to do is to click on the icon with the heading, for example, "Forgotten password", enter your email or phone number, and the system will send the user a newly generated password.

Another option for the first connection of the user to the system is the generation of a username and possibly also a password directly by the administrator or the superior role. After the first login, the user can change and save the password him/herself.

### User data editing

Each user connected using registered access (with a password) is entitled to edit the selected information according to his/her pre-set role.

A user with the set-up role "Dog owner" can edit data / attributes about his/her own dog. After clicking on an icon with the title, for example "My dog", he/she will see a list of all the dogs that he/she has and/or had. After clicking on the details of his/her dog, the user has the right to edit the registered data. An exception may be information from the health card, which can only be edited by a user named "Veterinarian". These are mainly newly acquired titles from dog shows or performance tests.

As far as other dogs and breeding establishments are concerned, the user can only see selected data, with no possibility to edit them.

### Logging of changes - checking changes to registered dog data

Due to the fact that not only can the dog owner edit the registered information about his/her own dog, but can also view any so-called logging of changes, the owner of the dog can see who, when and which data about his/her own dog has been changed.

### Overview of Figures in the Drawings

Fig. 1 shows a diagram listing the entities and their rights to access and handle data in the database by means of the system according to the invention and listing the superior and subordinate interrelationships of the individual user roles.

### Examples of the Embodiments

### Example 1

The method according to the invention, which is based on the attached scheme in Fig. 1, where:
- access to the data on dogs is available to the user through a dedicated system processing instructions from communication means involving the user's device, depending on the user's authorization level;
- wherein the system comprises two levels of authorization for processing instructions from the user, in particular to access data in the stored database and to enter these data into the database;
- where the first group of user roles with the highest level of authorization is entitled to enter and edit data in the database based on the authorization of the user role belonging to this group and where this group of roles is also authorized to ask questions and give answers through the system, which are recorded by means of communication tools in the database and sent directly to the designated users by means of communication means, depending on their user role;
- and where another group of user roles is authorized through the system only to ask questions and give answers, which are recorded by means of communication tools in the database and are sent directly to the designated users by means of communication means, depending on their user role;
- wherein there exist superior and subordinate relationships within user roles, where the superior user role authorizes requests from the user role being subordinate to it, and the subordinate user role raises authorization requests to the superior user role;
- where entity 1 responsible for the organization of relations at the international level, which is an international canine organization, has the exclusive position of superiority to entity 2 responsible for the organization of cynology at the national level, which is the national canine organization;
- and further, four user roles are not in a subordinate and/or superior relationship to any other user role, wherein this user role is:
   - entity 11 responsible for the care of animals which are not in the possession of the owner or whose owner is unknown, and
   - entity 12 responsible for the catching and/or reporting of animals which are not in the possession of the owner or whose owner is unknown, which is a shelter;
   and where one of these user roles without a subordinate and/or superior relationship belongs to the first group of user roles with the highest level of authorization, said user role being entity 10 responsible for veterinary care, which is a veterinarian;
- wherein the user role, which is in a relationship of superiority and subordination to other user roles and belongs to the first group of user roles with the highest level of authority, is:
   - entity 2 responsible for the organization of cynology at the national level, which is the national canine organization, and further
   - entity 3 responsible for providing advice in the field of dog breeding, which is a breeding advisor, and further
   - entity 4, which is a breeders' club.

### Example 2

The method according to Example 1, wherein the first group of user roles with the highest level of authorization preferably comprises:
- entity 2 responsible for the organization of cynology at the national level, which is the national canine organization;
- entity 3 responsible for providing advice in the field of dog breeding, which is a breeding advisor;
- entity 4, which is a breeders' club;
- entity 5, which is a dog owner;
- entity 6, which is a breeding establishment owner;
- entity 7 responsible for assessing dogs' appearance, which is a judge assessing dogs' appearance;
- entity 8 responsible for assessing dogs' performance tests, which is a performance test assessor;
- entity 9 responsible for monitoring and supervising compliance with veterinary regulations, which is the state represented by the state veterinary administration, and
- entity 10 responsible for the veterinary care, which is a veterinarian.

### Example 3

The method according to Example 1, where advantageously the group of user roles without the highest authorisation comprises:
- entity 11 responsible for the care of animals which are not in the possession of the owner or whose owner is unknown, which is a shelter, and
- entity 12 responsible for catching and/or reporting animals that are not in the possession of the owner or whose owner is unknown, which is the police, and it also contains entities 13 without determination of responsibility.

### Example 4

The method according to example 1, where the initial authorization of the user according to its user role is carried out by means of an electronic invitation provided by an authorized entity, sent to the user to its specified electronic mail address, and subsequent connection via a link in the electronic invitation by entering the registration in the pre-filled identification form of the data application, where the member adds its password to the pre-filled identification data of the member and is authorised, or the user is authorized by creating a user account by means of the application after personal verification of the user's identity by an authorized entity or by an entity authorized by this entity when, after the user's authorization, the login data to the application are made available to the user.

### Example 5

The method according to examples 2 and 3, where the entity 2 responsible for the organization of cynology at the national level is in a superior relationship to the entities that are:
- entities 3 responsible for providing advice in the field of dog breeding, which are breeding advisors;
- entities 4, which are breeders' clubs;
- entities 5, which are dog owners;
- entities 6, which are breeding establishment owners;
- entities 7 responsible for dog appearance assessing, which are judges assessing dogs' appearance;
- entities 8 responsible for assessing dogs' performance tests, which are performance test assessors;
wherein entity 2 is further in a subordinate relationship to entities which are:
- entity 1 responsible for the organization of relations at the international level, which is the International Canine Federation (FCI), and
- entities 9 responsible for monitoring and supervising compliance with veterinary regulations, which are the states represented by their state veterinary administrations.

### Example 6

The method according to examples 2 and 3, where entity 3 responsible for providing advice in the field of dog breeding is in a superior relationship to the entities that are:
- entities 4, which are dog owners;
- entities 6, which are breeding establishment owners;
wherein entity 3 is in a subordinate relationship at least to entities that are:
- entities 2 responsible for the organization of cynology at the national level, which are national canine organizations, and
- entities 4, which are breeders' clubs.

### Example 7

The method according to examples 2 and 3, where entity 5, which is a dog owner, is in a subordinate relationship to entities which are:
- entities 2 responsible for the organization of cynology at the national level, which are national canine organizations,
- entities 3, which are breeding advisors, and
- entities 9 responsible for monitoring and supervising compliance with veterinary regulations, which is the state represented by its state veterinary administration.

### Example 8

The method according to examples 2 and 3, where entity 6, which is a breeding establishment owner, is in a subordinate relationship to entities that are:
- entities 2 responsible for the organization of cynology at the national level, which are national canine organizations,
- entities 3, which are breeding advisors, and
- entities 9 responsible for monitoring and supervising compliance with veterinary regulations, which are the states represented by their state veterinary administrations.

### Example 9

The device for carrying out the method according to Examples 1 to 8 comprises:
- a remote data storage, which is a cloud data storage and a data server;
- mobile and non-portable computing devices such as mobile smartphones, tablets and personal computers; and
- chips which mark animals for the subsequent identification of their owner.

### Industrial utilisation

The invention is industrially applicable primarily to improve the breeding of all breeds of dogs and to speed up the collection and refinement of data on dogs for all canine organizations and breeders' clubs. Because the current way of sharing data on dogs within one country and across all countries, combined with paper pedigree certificates, does not allow a good quality selection of the ideal individual for mating, especially due to ignorance of genetic defects and diseases of dogs and their offspring and other important characteristics of dogs (dog shows, tests, etc.), one can assume interest from all states, international and national canine organizations, breeders' clubs, dog owners and breeding establishments in this technical solution.

### List of Reference Marks

1 - entity responsible for organizing relations at the international level;
2 - entity responsible for organizing relations at the national level;
3 - entity responsible for providing advice on dog breeding;
4 - entity that is a breeders' club;
5 - entity that is a dog owner;
6 - entity that is a breeding establishment owner;
7 - entity responsible for assessing the appearance of dogs;
8 - entity responsible for assessing performance tests;
9 - entity responsible for monitoring and supervising compliance with veterinary regulations;
10 - entity responsible for veterinary care;
11 - entity responsible for the care of animals which are not in the possession of their owner or whose owner is unknown;
12 - entity responsible for catching and/or reporting animals which are not in the possession of the owner or whose owner is unknown;
13 - other entities.

## Claims

1. A method for collecting data on dogs, accessing and handling these data, where the collection of data on dogs takes place by an authorized user entering relevant data according to its user role into a data application uploaded locally to that user's device and/or accessible via the user's device by means of a data network and stored on a remote data storage, wherein the data are uploaded online in essence in real time or after termination of the offline mode of the data application to the database in the remote data storage, **characterized in that**
- access to the data on dogs is available to the user by means of a dedicated system processing instructions from communication means involving the user's device, depending on the user's authorization level,
- wherein the system comprises at least two levels of authorization to process instructions from the user, in particular to access data stored in the database and to enter these data into the database,
- where the first group of user roles with the highest level of authorization is authorized to enter and/or edit data in the database based on the authorization of the user role belonging to this group, and is also authorized to ask questions and give answers through the system, which are recorded by communication tools in the database and/or are sent directly to designated users by communication means, depending on their user role,
- where another group of user roles is authorized by means of the system only to ask questions and give answers, which are recorded by means of communication tools in the database and/or are sent directly by means of communication means to designated users depending on their user role,
- wherein in the framework of user roles there are superior and subordinate relationships, where the superior user role usually authorizes the requirements of the user role subordinate to it and the subordinate user role raises requests for authorization to the superior user role,
- wherein at least one user role, usually entity (1) responsible for organizing relations at the international level, which is usually an international canine organization, has an exclusive position of superiority over at least one other user role, which is in a superior and/or subordinate relationship to other user roles;
- and furthermore at least two user roles are not in a subordinate and/or superior relationship to any other user role, where at least one user role without a subordinate and/or superior relationship belongs to the first group of user roles with the highest level of authorization,
- wherein at least one user role which is in a superior and subordinate relationship to other user roles belongs to the first group of user roles with the highest level of authorization.

2. The method according to claim 1, **characterized in that** the first group of user roles with the highest level of authorization includes at least one entity selected from the group of entities, where:
- at least one entity (2) is responsible for the organization of cynology at the national level;
- at least one entity (3) is responsible for providing advice in the field of dog breeding;
- at least one entity (4) is a breeders' club;
- at least one entity (5) is a dog owner;
- at least one entity (6) is the owner of a breeding establishment;
- at least one entity (7) is responsible for assessing the appearance of dogs;
- at least one entity (8) is responsible for assessing the performance tests of dogs;
- at least one entity (9) is responsible for monitoring and supervising compliance with veterinary regulations, and
- at least one entity (10) is responsible for veterinary care.

3. The method according to claim 1, **characterized in that** the group of user roles without the highest authorisation comprises at least one entity of the group of entities, where:
- at least one entity (11) is responsible for caring for animals that are not in the possession of their owner or whose owner is unknown;
- at least one entity (12) is responsible for catching and/or reporting animals that are not in the possession of their owner or whose owner is unknown, and alternatively it also comprises at least one entity (13 without determination of responsibility.

4. The method according to claim 1, **characterized in that** the initial authorization of the user according to its user role takes place by means of an electronic invitation from the authorized person sent to the user to its specified electronic mail address with subsequent connection via a link in the electronic invitation by entering the registration form of the data application, where the member adds his/her password in the pre-filled identification data of the member and is therefore authorised, or the user is authorized by creating a user account through the application after personal verification of the user's identity by an authorized person or by an entity authorised by the authorised person, where when the user is authorized, the user is provided with login data to the database.

5. The method according to claim 2 or 3, **characterized in that** entity (2) responsible for the organization of cynology at the national level is in a superior relationship to at least one entity of the group comprising:
- at least one entity (3) responsible for providing advice in the field of dog breeding;
- at least one entity (4), which is a breeders' club;
- at least one entity (5), which is a dog owner;
- at least one entity (6), which is a breeding establishment owner;
- at least one entity (7), which is responsible for assessment of the appearance of dogs;
- at least one entity (8,) which is responsible for assessment of the performance tests of dogs;
wherein entity (2) responsible for the organisation of relations at the international level is further in a subordinate relationship to at least one entity of the group comprising:
- at least one entity (1) responsible for the organization of relations at the international level, and
- at least one entity (9) responsible for monitoring and supervising compliance with veterinary regulations.

6. The method according to claim 2 or 3, **characterized in that** entity (3) responsible for providing advice in the field of the breeding of dogs is in a superior relationship to at least one entity from the group comprising:
- at least one entity (5), which is a dog owner a
- at least one entity (6), which is the owner of a breeding establishment;
wherein entity (3) responsible for providing advice in the field of the breeding of dogs is in a subordinate relationship to at least one entity from the group comprising:
- at least one entity (2) that is responsible for organization of cynology at the national level, and
- at least one entity (4), which is a breeders' club.

7. The method according to claim 2 or 3, **characterized in that** entity (5), which is a dog owner, is in a subordinate relationship to at least one entity of the group comprising:
- at least one entity (2) that is responsible for the organization of cynology at national level;
- at least one entity (3) that is responsible for providing advice in the field of dog breeding, and
- at least one entity (9) responsible for monitoring and supervising compliance with veterinary regulations.

8. The method according to claim 2 or 3, **characterized in that** entity (6), which is the owner of a breeding establishment, is in a subordinate relationship to at least one entity from the group comprising:
- at least one entity (2)that is responsible for the organization of cynology at the national level;
- at least one entity (3) that is responsible for providing advice in the field of dog breeding, and
- at least one entity (9) that is responsible for monitoring and supervising compliance with veterinary regulations.

9. A device for carrying out the method according to any one of claims 1 to 8, **characterized in that** it comprises the following elements:
- aremote data storage, in particular a cloud data storage and/or a data server;
- mobile and/or non-portable computing devices, including in particular a smartphone, tablet and personal computer;
- a chip identifying animals for the subsequent identification of their owner.
